# EUROPÄISCHE PATENTSCHRIFT

(11) **EP 2 281 532 B1**
(45) Veröffentlichungstag und Bekanntmachung des Hinweises auf die Patenterteilung: **07.09.2016**
(21) Anmeldenummer: 10007817.9
(22) Anmeldetag: 28.07.2010
(51) Int. Cl.: A61F 2/46, B01F 15/02, B01F 15/00

(54) **Knochenzementsystem**
Bone cement system
Système de ciment osseux

(30) Priorität: 28.07.2009 DE 102009035067
(43) Veröffentlichungstag der Anmeldung: 09.02.2011
(73) Patentinhaber: Heraeus Medical GmbH, 61273 Wehrheim (DE)
(72) Erfinder: Vogt, Sebastian, Dr., 99092 Erfurt (DE); Stöckli, Rochus, 6374 Buochs (CH); Büchner, Hubert, Dr., 90491 Nürnberg (DE)
(74) Vertreter: Heraeus IP

(56) Entgegenhaltungen:
- EP-A2- 1 005 900
- WO-A1-00/35506
- WO-A1-94/26403
- WO-A2-2005/018830
- DE-A1- 19 532 015
- US-A1- 2009 057 168

## Beschreibung

Die Erfindung betrifft ein Knochenzementsystem gemäß dem Oberbegriff des Patentanspruchs 1 mit einer Mischvorrichtung zum Mischen und Austragen von Knochenzement, einem Vorratsbehälter für einen Monomer und einem Leitungsmittel, wobei die Mischvorrichtung einen Mischzylinder aufweist, der Mischzylinder ein Knochenzementpulver lagert, mittels des Leitungsmittels der Monomer aus dem Vorratsbehälter in den Mischzylinder leitbar ist, zwischen dem Vorratsbehälter und der Mischvorrichtung ein Siebelement angeordnet ist, um ein Einströmen des Knochenzementpulvers aus dem Mischzylinder in das Leitungsmittel zu verhindern, die Mischvorrichtung eine Austragsöffnung aufweist, um einen aus dem Knochenzementpulver und dem Monomer gemischten Knochenzement auszutragen.

PMMA-Knochenzemente sind seit Jahrzehnten bekannt und gehen auf die grundlegenden Arbeiten von Sir Chamley zurück (Charnley, J.: Anchorage of the femoral head prosthesis of the shaft of the femur. J. Bone Joint Surg. 42 (1960) 28-30.). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Bei dem Vermischen der Pulverkomponente mit der Monomerkomponente reagiert der Aktivator N,N-Dimethyl-p-toluidin, mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs, bis dieser erstarrt.

Polymethylmethacrylat-Knochenzemente können in geeigneten Mischbechem mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzementes - auch als Zement bezeichnet - verursachen können. Aus diesem Grund wird das Vermischen von Knochenzementpulver mit der Monomerflüssigkeit in Vakuum-Mischsystemen bevorzugt, weil durch Mischen im Vakuum Lufteinschlüsse aus dem Zementteig weitgehend entfernt werden und damit eine optimale Zementqualität erreicht wird (Breusch SJ et al.: Der Stand der Zementiertechnik in Deutschland. Z Orthop. 1999, 137: 101-07). Im Vakuum gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US6033905 A, US562184 A, US4871263 A, US4973168 A, US5100241 A, WO99167015 A1, EP1020167 A2, US5586821 A, EP1016452A2, DE3640279 A1, WO94/26403 A1, EP0692229 A1, EP1005901 A2, US5344232 A.

Eine Weiterentwicklung stellen Zementiersysteme dar, in denen sowohl das Zementpulver als auch die Monomerflüssigkeit bereits in separaten Kompartimenten der Mischsysteme verpackt sind und erst unmittelbar vor der Zementapplikation im Zementiersystem miteinander vermischt werden (US5997544 A, EP0692229 A1, US6709149 B1). Problematisch ist bei allen diesen Systemen die Überführung der Monomerflüssigkeit in das Zementpulver und die vollständige Vermischung dieser beiden Komponenten, damit ein homogener Zementteig erhalten wird, der insbesondere keine Nester an nicht von der Monomerflüssigkeit benetztem Zementpulver enthalten darf. Bei einem gegenwärtig in Europa auf dem Markt befindlichen Mischsystem wird durch Röhren, die seitlich im unteren Teil der Kartusche angebracht sind, welche die Kartuschenwand durchstoßen, die Monomerflüssigkeit ungefähr in der Mitte des Zementpulvers durch Einwirkung von Vakuum in das Zementpulver eingebracht. Dabei ist keine Mischvorrichtung an den Röhren vorgesehen, die ein Eindringen des Zementpulvers während der Lagerung des Mischsystems in die Röhren verhindern kann. Eine Verstopfung der Röhren durch Zementpulver ist nicht völlig auszuschließen.

Die DE 698 12 726 T2 zeigt ein weiteres Mischsystem zum Mischen und Austragen von Knochenzement. Das Mischsystem weist einen Mischzylinder aufweist, wobei zwischen dem Vorratsbehälter und der Mischvorrichtung ein Siebelement angeordnet ist, um ein Einströmen des Knochenzementpulvers aus dem Mischzylinder in das Leitungsmittel zu verhindern. Als Nachteilig hat es sich bei solchen Mischsystem herausgestellt, dass eine gleichmäßige und schnelle Durchmischung des Monomers mit dem Knochenzementpulver nicht immer sichergestellt werden kann.

Eine andere Variante wurde In EP 1 140 234 B1 offen gelegt. Bei diesem Mischsystem wird die Monomerflüssigkeit mit Hilfe eines Vakuums durch das gesamte Zementpulver gesaugt. Der grundsätzliche Ansatz, die Monomerflüssigkeit durch das gesamte Zementpulver zu saugen, um damit eine optimale Vermischung zu erreichen und um Nester aus nicht benetztem Zementpulver zu vermeiden, ist nur machbar, wenn ein Zementpulver verwendet wird, das sehr langsam nach Benetzung durch die Monomerflüssigkeit anquillt. Das bedeutet, dass die gegenwärtig in der Endoprothetik am häufigsten eingesetzten hoch-viskosen und mittel-viskosen PMMA-Knochenzemente nicht oder nur erschwert eingesetzt werden können, weil das Zementpulver dieser Zemente nach Benetzung durch die Monomerflüssigkeit sofort anquillt und einen Teig bildet, der eine weitere Durchdringung des Zementpulvers mit der Monomerflüssigkeit erschwert oder sogar unmöglich macht.

Die Aufgabe der Erfindung besteht darin, ein Knochenzementsystem zu entwickeln, das die vorgenannten Nachteile vermeidet, insbesondere gegen eine Verstopfung durch Zementpulver geschützt ist.

Zur Lösung dieser Aufgabe wird ein Knochenzementsystem mit den Merkmalen des Anspruchs 1 vorgeschlagen. In den abhängigen Ansprüchen sind bevorzugte Weiterbildungen ausgeführt.

Es ist erfindungsgemäß vorgesehen, die Austragsöffnung eine Blende mit mindestens einer Durchgangsöffnung aufweist, und ein Verhältnis einer Fläche der Durchgangsöffnung zur Fläche des Siebelementes mindestens 1 zu 3 ist und ein Abstand zwischen der Blende und dem Siebelement mindestens 1 mm ist.

Die Erfindung ermöglicht es, Monomerflüssigkeit von unten in das Knochenzementpulver - auch als Zementpulver bezeichnet - so einzuspritzen, dass eine Verklebung des Einspritzsystems vermieden wird und eine möglichst vollständige Durchmischung des Zementpulvers erreicht werden kann. Das Knochenzementsystem darf nicht verkleben, weil dadurch der vollständige Monomertransfer aus dem Monomervorratsbehälter in das Zementpulver unmöglich wird. Ein unvollständiger Monomertransfer hätte zur Folge, dass nur ein Teil des vorgesehen Monomers mit dem Zementpulver einen Teig bilden würde. Der entstehende Teig wäre dadurch viskoser und nach der Aushärtung hätte der Knochenzement - auch als Zement bezeichnet - nicht vorhersehbare veränderte mechanische Eigenschaften im Vergleich zum korrekt angemischten Zement, der mit einem vorbestimmten Verhältnis von Monomerflüssigkeit zu Zementpulver hergestellt wird. Das Knochenzementsystem funktioniert in der Weise, dass durch Anlegen von Vakuum gegenüber der Durchgangsöffnung ein Unterdruck in der Zufuhröffnung entsteht und zuerst die im System enthaltende Restluft und danach die Monomerflüssigkeit in den Zwischenraum gesaugt wird. Die zuerst auftretende Luft tritt durch das Siebelement und reißt das im Zwischenraum vorhandene Zementpulver durch die Durchgangsöffnung mit In Richtung des Mischzylinders. Der Zwischenraum enthält dadurch kein Zementpulver und ist leer. Danach wird die Monomerflüssigkeit durch das Siebelement gesaugt. Somit ermöglicht das Siebelement, dass das Knochenzementpulver nicht In das Leitungsmittel einströmt und dieses bei Kontakt mit der Monomerflüssigkeit verstopft. Erfindungsgemäß trennt also das Siebelement das Leitungsmittel und den Mischzylinder derart, dass kein Knochenzementpulver in das Leitungsmittel einströmen kann.

Eine vorteilhafte Ausgestaltungsvariante des erfindungsgemäßen Knochenzementsystems zeichnet sich dadurch aus, dass die Mischvorrichtung eine Austragsöffnung aufweist, um einen aus dem Knochenzementpulver und dem Monomer gemischten Knochenzement auszutragen. Nach Durchmischung des Knochenzementpulvers und des Monomers bildet sich ein Knochenzement. Dieser muss vor seiner Erstarrung aus dem Knochenzementsystem ausgetragen und vorzugsweise in den Patienten implantiert werden. Dazu hat es sich als vorteilhaft erwiesen, wenn eine Austragsöffnung vorhanden ist, durch welche der Knochenzement aus dem Mischzylinder herausgepresst werden kann. Vorteilhafterweise ist die Austragsöffnung trichterartig ausgestaltet. Eine weitere vorteilhafte Ausgestaltung zeichnet sich dadurch aus, dass das Siebelement in einem Zwischenraum gelagert ist, der mit der Austragsöffnung der Mischvorrichtung verbindbar ist und in den das Leitungsmittel endet. In dem Zwischenraum endet sowohl die Austragsöffnung der Mischvorrichtung, als auch das Leitungsmittel. Durch den Zwischenraum strömt folglich der Monomer aus dem Vorratsbehälter in den Mischzylinder. Innerhalb dieses Zwischenraums ist das erfindungsgemäße Siebelement angeordnet. Diese Ausgestaltung weist den Vorteil auf, dass das Siebelement nicht in jener Austragsöffnung angeordnet ist, durch die später der gemischte Knochenzement ausgetragen wird. Allerdings verhindert das Siebelement ein Einströmen des Knochenzementpulvers aus dem Mischzylinder in das Leitungsmittel.

Eine weitere vorteilhafte Ausgestaltung zeichnet sich dadurch aus, dass das Siebelement eine Maschenweite von kleiner als 30 µm hat, insbesondere eine Maschenweite zwischen 5 µm und 25 µm. Diese Maschenweite stellt zum einen sicher, dass das Knochenzementpulver nicht aus der Mischvorrichtung In das Leitungsmittel hineinströmen kann. Andererseits wird der Fluss des insbesondere liquiden Monomers aus dem Vorratsbehälter in den Mischzylinder nur unwesentlich durch die Maschen des Siebelements behindert. Somit stellt ein Siebelement mit einer Maschenweite zwischen 30 µm und 5 µm ein nur einseitig geöffnetes Verbindungselement zwischen dem Vorratsbehälter und der Mischvorrichtung dar.

In einer weiteren vorteilhaften Ausgestaltung weist die Austragsöffnung eine Blende mit zumindest mindestens einer Durchgangsöffnung auf. Die Blende ist vorteilhafterweise zwischen der Mischvorrichtung, insbesondere der Austragsöffnung, und dem Siebelement angeordnet Dabei bestimmt die Austragsöffnung die Menge des Monomers, der in den Mischzylinder innehalb einer Zeiteinheit einfließen kann, Vorteilhafterweise ist die Fläche der Durchgangsöffnung kleiner als die Fläche des Siebelements. Vorteilhafterweise ist das Siebelement und die Blende Teil eines Zwischenraums. An diesen Zwischenraum schließt sich einerseits das Leitungsmittel des Vorratsbehälters und andererseits die Austragsöffnung der Mischvorrichtung an. Der Zwischenraum ist derart ausgestaltet, dass er etwa mittig das Siebelement lagert, welches im Wesentlichen parallel zu der Blende angeordnet ist. Bei Lagerung und Transport des Polymethylmethacrylat-Knochenzementmischsystems verhindert das Siebelement den Durchtritt des Zementpulvers in Richtung der Zufuhröffnung. Das Knochenzementsystem funktioniert in der Weise, dass durch Anlegen von Vakuum gegenüber der Durchgangsöffnung ein Unterdruck in der Zufuhröffnung entsteht und zuerst die im System enthaltende Restluft und danach die Monomerflüssigkeit in den Zwischenraum gesaugt wird. Die zuerst auftretende Luft tritt durch das Siebelement und reißt das im Zwischenraum vorhandene Zementpulver durch die Durchgangsöffnung mit in Richtung des Mischzylinders. Der Zwischenraum enthält dadurch kein Zementpulver und ist leer. Danach wird die Monomerflüssigkeit durch das Siebelement gesaugt. Aufgrund der geringen Maschenweite des Siebelements kommt es dadurch zu einer deutlichen Verminderung der Fließgeschwindigkeit. Diesem Geschwindigkeitsverlust wird durch eine entsprechend große Siebfläche begegnet. Das bedeutet, auch bei langsamerem Durchtritt der Flüssigkeit durch das Siebelement wird aufgrund der großen Fläche trotz des Strömungswiderstandes der Volumenstrom nicht gebremst. Unter dem Begriff Volumenstrom wird das Volumen Monomerflüssigkeit pro Zeiteinheit verstanden, das aus der Zuführöffnung heraustritt. Danach sammelt sich die Monomerflüssigkeit im Zwischenraum und bewegt sich in Richtung der Durchgangsöffnung.

Das erfindungsgemäßen Knochenzementsystems zeichnet sich dadurch aus, dass ein Verhältnis einer Fläche der Durchgangsöffnung zur Fläche des Siebelementes mindestens 1 zu 3 ist bevorzugt, dass das Verhältnis der Fläche der Durchgangsöffnung zur Fläche des Siebelementes zwischen 1 zu 4 und 1 zu 20 ist. Durch die geringe Fläche der Durchgangsöffnung gegenüber der Fläche des Siebelements wird die Flüssigkeit in der Austrittsöffnung beschleunigt, damit der Volumenstrom konstant bleibt, Das bedeutet, es entsteht ein Strahl der Monomerflüssigkeit, der in das über der Durchgangsöffnung befindliche Zementpulver gespritzt wird. Der Strahl des Monomers weitet sich mit zunehmender Flugweite trichterförmig aus. Durch die hohe Geschwindigkeit des Monomerstrahls kann das Monomer in einer solch kurzen Zeit durch das Zementpulver gelangen, bevor das Zementpulver in größerem Umfang gequollen ist. Schon angequollene Knochenzementpulverpartikel werden durch den Monomerstrahl verdrängt.

Das Vermeiden eines Einströmens des Knochenzementpulvers aus dem Mischzylinder in das Leitungsmittel wird dadurch gefördert, dass ein Abstand zwischen der Blende und dem Siebelement mindestens 1 mm ist, bevorzugt, dass der Abstand zwischen der Blende und dem Siebelement zwischen 2 mm bis 10 mm ist. Die aufgeführten Werte haben sich bei umfangreichen Messungen überraschenderweise als besonders bevorzugt herausgestellt, um sicherzustellen, dass der Monomer einerseits beim Eintritt in den Mischzylinder mögliche Knochenzementpulverreste aus dem Zwischenraum in den Mischzylinder mitreißt und andererseits den Zwischenraum nicht derart groß gestaltet, dass die Menge sich dort ablagernder Knochenzementpulverreste möglichst gering ist.

Je nach verwendetem Knochenzementpulver hat es sich als vorteilhaft herausgestellt, wenn die Durchgangsöffnung kreisförmig, oval, sternförmig oder schlitzförmig ausgebildet ist, insbesondere, dass die Blende trichterförmig verläuft. Die Formen der Durchgangsöffnung können das Strömungsverhalten des Monomers in das Knochenzementpulver steuern. Insbesondere stern- oder schlitzförmige Durchgangsöffnungen sorgen für eine trichterförmige Aufspaltung des Strahles der Monomerflüssigkeit, der durch die Blende in den Mischzylinder eintritt. Zusätzlich kann die Blende trichterförmig verlaufen. In diesem Fall wirkt die Blende wie eine Venturi-Düse und sorgt für eine zusätzliche Beschleunigung der Monomerflüssigkeit, die aus dem Zwischenraum in den Mischzylinder einströmt.

Um eine möglichst gleichmäßige Vermischung des Monomers mit dem Knochenzementpulver zu erzielen, hat es sich als vorteilhaft herausgestellt, wenn in dem Mischzylinder ein Mischkolben angeordnet ist, wobei der Mischkolben mittels einer an einem ersten Zylinderende gedichtet herausgeführten Betätigungsstange axial bewegbar ist, Vorteilhafterweise liegt das erste Zylinderende einem zweiten Zylinderende entgegengesetzt, wobei das zweite Zylinderende die Austragsöffnung aufweist. Somit kann das In den Mischzylinder einströmende Monomer mittels des Mischkolbens bzw. der Betätigungsstange noch weiter in den Mischzylinder gezogen werden, um für eine gleichmäßige Vermischung des Zementpulvers mit dem Monomer zu sorgen.

Eine Besonderheit der erfindungsgemäßen Mischvorrichtung besteht darin, dass das Kolbensystem axial in den Mischzylinder eindrückbar ist, um einen aus dem Knochenzementpulver und dem Binder, insbesondere dem Monomer, gemischten Knochenzement, durch eine Austragsöffnung auszutragen. Die Austragsöffnung liegt an einem zweiten Zylinderende des Mischkolbens. Das zweite Zylinderende ist dem ersten Zylinderende entgegengesetzt. Beim Austragen wird das Kolbensystem aus Richtung des ersten Zylinderendes in Richtung des zweiten Zylinderendes gedrückt und presst dabei den fertig gemischten Knochenzement durch die Austragsöffnung heraus. In einer vorteilhaften Ausgestaltung weist die Austragsöffnung ein Anschlussmittel, insbesondere ein Anschlussgewinde, auf. Dieses Anschlussgewinde kann genutzt werden, um den Mischzylinder in das Knochenzementsystem einzuschrauben und/oder den Mischzylinder an ein Schlauchsystem anzuschließen, Ober das der fertige Knochenzement in den Knochen eingebracht werden kann. Für diese Tätigkeit kann eine Auspresspistole genutzt werden, in die der Mischzylinder eingespannt wird. Zur einfachen Benutzung der Auspresspistole kann die Betätigungsstange eine Sollbruchstelle aufweisen, so dass ein Abbrechen der Betätigungsstange an einer definierten Stelle möglich ist. Zum Austragen des fertig gemischten Knochenzements wird die Betätigungsstange in Richtung des Kolbensystems gezogen, bis der Mischkolben an dem Kolbensystem anliegt. Durch das dann erfolgende Abtrennen der Betätigungsstange kann das Kolbensystem mit dem davor anliegenden Mischkolben in den Mischzylinder eingepresst werden.

In einer vorteilhaften Ausgestaltung weist die Austragsöffnung ein Anschlussmittel, insbesondere ein Anschlussgewinde, auf. Dieses Anschlussgewinde kann genutzt werden, um den Mischzylinder in das noch zu beschreibende Knochenzementsystem einzuschrauben und/oder den Mischzylinder an ein Schlauchsystem anzuschließen, über das der fertige Knochenzement in den Knochen eingebracht werden kann. Für diese Tätigkeit kann eine Auspresspistole genutzt werden, in die der Mischzylinder eingespannt wird. Zur einfachen Benutzung der Auspresspistole kann die Betätigungsstange eine Sollbruchstelle aufweisen, so dass ein Abbrechen der Betätigungsstange an einer definierten Stelle möglich ist. Zum Austragen des fertig gemischten Knochenzements wird die Betätigungsstange in Richtung des Kolbensystems gezogen, bis der Mischkolben an dem Kolbensystem anliegt. Durch das dann erfolgende Abtrennen der Betätigungsstange kann das Kolbensystem mit dem davor anliegenden Mischkolben in den Mischzylinder eingepresst werden. Weiterhin ist es vorteilhaft, wenn das Vorratselement einen Vorratsbehälter für den Monomer lagert. Zur Herstellung des Knochenzements muss der Monomer in das Knochenzementpulver eingebracht werden. Nach einer gewissen Zeitspanne härtet der Knochenzement dann aus. Offensichtlich kann der Knochenzement somit nicht austragsfertig in der Vorrichtung ausgeliefert werden. Es ist somit notwendig, dass das Knochenzementpulver und der Monomer bis kurz vor dem Austragen des Knochenzements getrennt gelagert werden müssen. Somit bietet es sich an, wenn das Vorratselement einen Vorratsbehälter für den Monomer aufweist. Als leicht zu desinfizieren haben sich insbesondere Glasbehälter herausgestellt, die als Vorratsbehälter für den Binder, insbesondere den Monomer, genutzt werden. Um einen Zufluss des Monomers zu steuern, kann das Vorratselement ein Ventilmittel aufweisen. Dieses Ventilmittel steuert und/oder löst den Zufluss des Monomers aus dem Vorratsbehälter in die erfindungsgemäße Vorrichtung aus.

Eine vorteilhafte Ausgestaltung des erfindungsgemäßen Knochenzementsystems zeichnet sich dadurch aus, dass das Knochenzementsystem ein Basiselement aufweist, wobei das Basiselement die Mischvorrichtung und den Vorratsbehälter lagert. Das Basiselement dient somit als Plattform sowohl für die erfindungsgemäße Mischvorrichtung, als auch das Vorratselement für den Binder. Als eine Art Fundament des Knochenzementsystems können die erfindungsgemäße Mischvorrichtung und das Vorratselement an und/oder auf dem Basiselement angeordnet sein. Eine vorteilhafte Ausgestaltung des erfindungsgemäßen Knochenzementsystems zeichnet sich dadurch aus, dass das Basiselement ein Koppelmittel aufweist für eine kraft- und/oder formschlüssige Verbindung mit der Mischvorrichtung, insbesondere einer Austragsöffnung der Mischvorrichtung. Da die erfindungsgemäße Mischvorrichtung auch zum Austragen des Knochenzements genutzt werden soll, ist es vorteilhaft, wenn die Mischvorrichtung reversibel von dem Basiselement trennbar ist. Dies kann durch das erfindungsgemäße Koppelement erreicht werden. Vorteilhafterweise handelt es sich bei dem Koppelelement um ein Gewinde, auf welches die Austragsöffnung der Mischvorrichtung aufgeschraubt werden kann. So ist eine sichere Verbindung zwischen dem Basiselement und der Mischvorrichtung gewährleistet.

In einer weiteren vorteilhaften Ausgestaltung kann das Basiselement das Leitungsmittel lagern. In diesem Falle durchläuft das Leitungsmittel das Basiselement. Auch der Zwischenraum kann In dem Basiselement angeordnet werden. Ober das Anschlussmittel ist eine Verbindung des Zwischenraumes und damit des Leitungsmittel mit dem Mischzylinder möglich. Erfindungsgemäß ist dabei Innerhalb des Zwischenraumes das Siebelement angeordnet, das ein Einströmen des Knochenzementpulvers aus dem Mischzylinder in das Leitungsmittel verhindert.

Weitere Maßnahmen und Vorteile der Erfindung ergeben sich aus den Ansprüchen, der nachfolgenden Beschreibung und den Zeichnungen. In den Zeichnungen ist die Erfindung in mehreren Ausführungsbeispielen dargestellt. Es zeigen:
- Fig. 1: eine schematische Schnittzeichnung eines erfindungsgemäßen Knochen zementsystems,
- Fig. 2: eine schematische Schnittzeichnung einer erfindungsgemäßen Mischvorrichtung und
- Fig. 3: eine schematische Schnittzeichnung einer Austragsöffnung der Mischvor richtung.

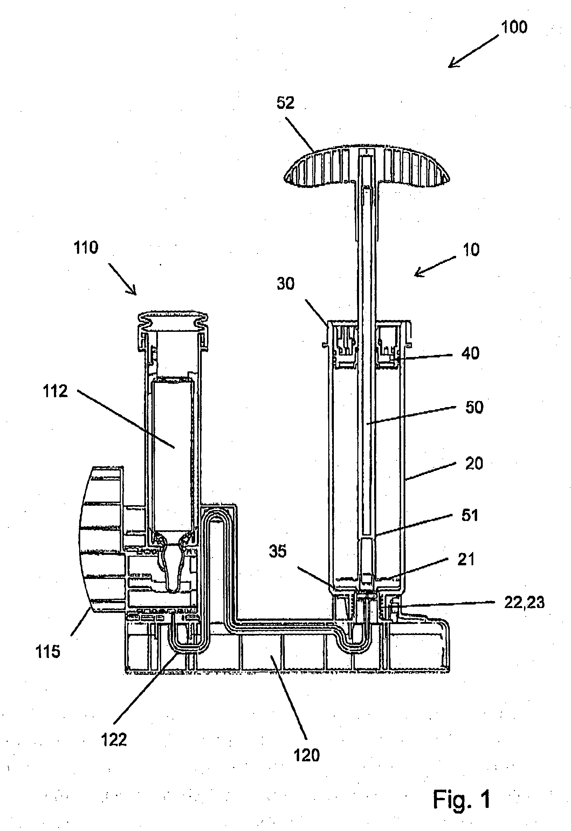

In Figur 1 ist ein erfindungsgemäßes Knochenzementsystem 100 dargestellt. Das Knochenzementsystem 100 weist eine Mischvorrichtung 10 zum Mischen und Austragen von Knochenzement auf. Diese Mischvorrichtung 10 ist in dem dargestellten Ausführungsbeispiel gelagert auf einem Basiselement 120. Jenes Basiselement 120 trägt auch ein Vorratselement 110 für einen Monomer. Das Knochenzementsystem 100 dient zum Mischen des Knochenzements. Dazu wird Knochenzementpulver in einen Mischzylinder 20 der Mischvorrichtung 10 eingefüllt. Jenes Knochenzementpulver kann dann mit dem Monomer vermischt werden, um Knochenzement zu bilden. Wie die Figur 1 verdeutlicht, ist Teil des Knochenzementsystems das Vorratselement 110. Das Vorratselement 110 lagert einen Vorratsbehälter 120 für den Monomer. Über ein Ventilmittel 115 kann ein Ausfluss des Monomers aus dem Vorratsbehälter 112 gesteuert und/oder ausgelöst werden. Vorteilhafterweise handelt es sich bei dem Vorratsbehälter 112 um einen Glasbehälter, der im Kopfbereich durch das Ventilmittel 115 geöffnet wird. Der Monomer fließt dann durch ein Leitungsmittel 122 von dem Vorratsbehälter 112 in den Mischzylinder 20. Das Überströmen des Monomers wird dadurch verstärkt, dass im Mischzylinder 20 ein Unterdruck herrscht. Mittels der Betätigungsstange und des Mischkolbens 21 ist dann ein leichtes und einfaches Vermischen des Knochenzementpulvers und des Monomers möglich. Nach fertiger Mischung kann die Mischvorrichtung 10 von dem Basiselement 120 abgeschraubt werden. Dazu weist das Basiselement 120 ein Koppelmittel 121 auf, welches mit einem Anschlussmittel 22 des Mischkolbens zusammenwirkt. Nach Trennung der Mischvorrichtung 10 von dem Basiselement 120 wird die Betätigungsstange 50 axial derart verschoben, dass der Mischkolben 21 am Kolbensystem 40 zum Liegen kommt. Anschließend lässt sich die Betätigungsstange an der Sollbruchstelle 51 abknicken. Die Mischvorrichtung 10 kann nunmehr in eine Zementierpistole integriert werden. Durch Betätigung der Zementierpistole wird eine Zahnstange mit Teller in Richtung des Kolbensystems 40 bewegt. Das Kolbensystem 40 wird zum Austragen des Knochenzements genutzt. Dazu ist das Kolbensystem 40 axial beweglich ausgestaltet und axial in den Mischzylinder 20 einrückbar. So wird erreicht, dass der aus Knochenzementpulver und dem Monomer gemischte Knochenzement durch eine Austragsöffnung 23 ausgetragen werden kann.

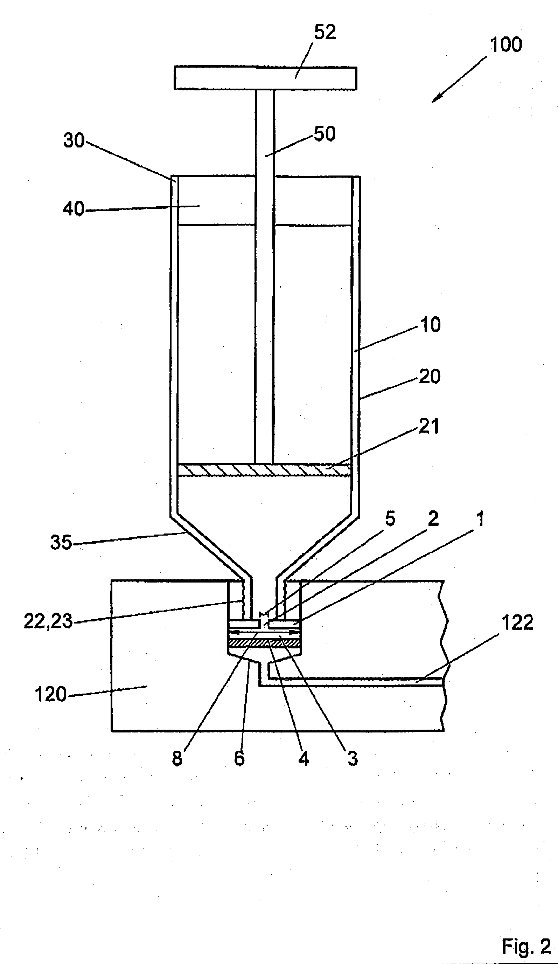

Im Stand der Technik sind Knochenzementsysteme bekannt, bei denen die Monomerflüssigkeit in Behältern seitlich des Mischzylinders gelagert wird. Durch Röhren wird die Monomerflüssigkeit ungefähr in der Mitte des in dem Mischzylinder angeordneten Zementpulvers eingebracht. Als nachteilig hat es sich herausgestellt, dass durch die Anordnung und Ausgestaltung der Röhren eine Verstopfung dieser durch Zementpulver nicht Völlig auszuschließen ist. Jenes kann zur Folge haben, dass die zugeführte Menge des Monomers nur unzureichend ist und sich dadurch ein nicht homogener Knochenzementbereich ergibt. Um diesen Nachteil zu überwinden, weist das erfindungsgemäße Knochenzementsystem 100 ein Siebelement 4 auf, wie es in der Figur 2 dargestellt ist. Diese Figur 2 ist eine Schnittzeichnung analog zu jener aus Figur 1, wobei die Merkmale im Bereich der Austragsöffnung 23 detaillierter dargestellt werden. Wie zu erkennen ist, lagert das Basiselement 120 den Mischzylinder 20 der Mischvorrichtung 10. Über ein Anschlussmittel, hier ein Gewinde, ist der Mischzylinder 20 mit dem Basiselement 120 verbunden. Unterhalb der Austragsöffnung 23 befindet sich in dem Basiselement eine Blende 1, die zumindest eine Durchgangsöffnung 2 aufweist. Wie zu erkennen ist, weist die Durchgangsöffnung eine Breite und damit auch eine Fläche 5 auf, die deutlich geringer ist als die Fläche des darunter angeordneten Siebelements 4. Dessen Breite ist mit dem Bezugszeichen 8 versehen. Vorteilhafterweise ist die Fläche der Durchgangsöffnung ein Viertel bis ein Zwanzigstel der Fläche des Siebelements 4. Durch das Siebelement 4 wird verhindert, dass bei der Lagerung und beim Transport Knochenzement aus dem Mischzylinder 20 in das Leltungsmittel 122 eindringt und dieses verstopfen kann. Soll nun der fertige Knochenzement gemischt werden, wird das Knochenzementsystem an ein Vakuumelement angeschlossen. Dadurch entsteht in dem Mischzylinder 20 sowie einem Zwischenraum 3, der das Siebelement 4 lagert, ein Unterdruck. Dieser Unterdruck saugt Restluft aus dem Zwischenraum 3 in Richtung des Vakuumanschlusses, der im Allgemeinen im Bereich eines ersten Zylinderendes 30 angeordnet ist. Durch dieses Ansaugen der Restluft wird noch im Zwischenraum 3 vorhandenes Zementpulver durch die Durchgangsöffnung 2 der Blende 1 in Richtung des Mischzylinders 20 gesaugt. Im Anschluss daran kann die Monomerflüssigkeit durch das Siebelement 4 gesaugt werden. Vorteilhafterweise weist dabei das Siebelement 4 eine Maschenweite von weniger als 30 µm auf. Aufgrund dieser geringen Maschenweite des Siebelements 4 kommt es zu einer Verminderung der Fließgeschwindigkeit des Monomers. Um dennoch einen gleichmäßigen Durchtritt der Flüssigkeit des Monomers in den Mischzylinder 20 zu erzielen, muss die Größe der Siebfläche 4 entsprechend angepasst sein. Das Verhältnis der Fläche 5 der Durchgangsöffnung 2 zur Fläche 8 des Siebelements 4 bestimmt somit auch das Volumen der in einer Zeiteinheit in den Mischzylinder 20 eingesaugten Monomerflüssigkeit. Durch die erfindungsgemäße Nutzung des Siebelements 4 ist es somit möglich, einen gleichmäßigen Einstrom des Monomers in den Mischzylinder 20 sicherzustellen und gleichzeitig ein Verstopfen des Leitungsmittels 122 durch Knochenzementpulver zu verhindern.

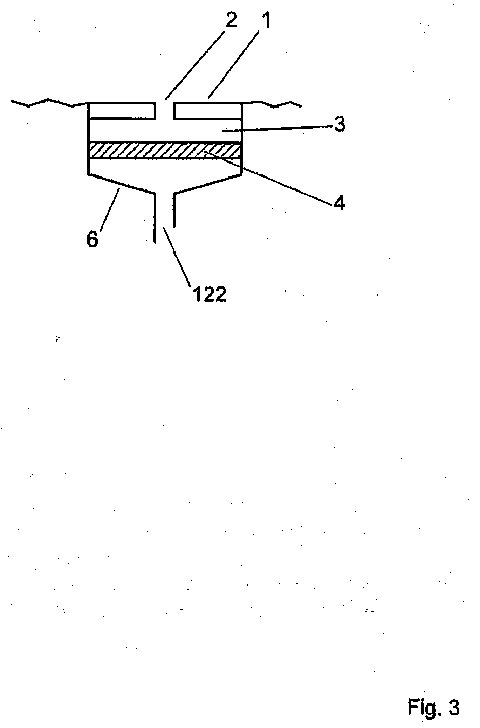

Die Figur 3 zeigt eine weitere Ausschnittsvergrößerung des Zwischenraums 3 mit dem integrierten Siebelement 4, das dazu dient, ein Einströmen des Knochenzementpulvers aus dem Mischzylinder 2 durch die Durchgangsöffnung 3 in das Leitungsmittel 122 zu verhindern. Der Zwischenraum 3 weist einen V-förmig gestalteten Boden 6 auf, der die Wirkung einer Düse aufweist, für das aus dem Leitungsmittel 122 ausströmende Monomer. Oberhalb dieser Austrittsöffnung des Leitungsmittels 122 ist das Siebelement 4 angeordnet. Bei diesem Siebelement kann es sich um eine gestanzte, gewebte oder gewirkte Struktur handeln, die aus Metallen, Kunststoffen und/oder Kombinationen von beiden zusammengesetzt ist.

### Bezugszeichen

- 1: Blende
- 2: Durchgangsöffnung
- 3: Zwischenraum
- 4: Siebelement
- 5: Breite der Durchgangsöffnung
- 6: Boden des Zwischenraums
- 8: Breite des Siebelements
- 10: Mischvorrichtung

- 20: Mischzylinder
- 21: Mischkolben
- 22: Anschlussmittel
- 23: Austragsöffnung

- 30: Erstes Zylinderende
- 35: Zweites Zylinderende

- 40: Kolbensystem

- 50: Betätigungsstange
- 51: Sollbruchstelle
- 52: Handgriff

- 100: Knochenzementsystem

- 110: Vorratselement
- 112: Vorratsbehälter
- 115: Ventilmittel

- 120: Basiselement
- 121: Koppelmittel
- 122: Leitungsmittel

## Patentansprüche

1. Knochenzementsystem (100) mit einer Mischvorrichtung (10) zum Mischen und Austragen von Knochenzement, einem Vorratsbehälter (112) für einen Monomer und einem Leitungsmittel (122), wobei
die Mischvorrichtung (10) einen Mischzylinder (20) aufweist,
der Mischzylinder (20) ein Knochenzementpulver lagert,
mittels des Leitungsmittels (122) der Monomer aus dem Vorratsbehälter (112) in den Mischzylinder (20) leitbar ist,
zwischen dem Vorratsbehälter (112) und der Mischvorrichtung (10) ein Siebelement (4) angeordnet ist, um ein Einströmen des Knochenzementpulvers aus dem Mischzylinder (20) in das Leitungsmittel (122) zu verhindern,
die Mischvorrichtung (10) eine Austragsöffnung (23) aufweist, um einen aus dem Knochenzementpulver und dem Monomer gemischten Knochenzement auszutragen,
**dadurch gekennzeichnet, dass**
die Austragsöffnung (23) eine Blende (1) mit mindestens einer Durchgangsöffnung (2) aufweist, und
ein Verhältnis einer Fläche der Durchgangsöffnung (2) zur Fläche des Siebelementes (4) mindestens 1 zu 3 ist und ein Abstand zwischen der Blende (1) und dem Siebelement (4) mindestens 1 mm ist.

2. Knochenzementsystem (100) nach Anspruch 1, **dadurch gekennzeichnet, dass** das Siebelement (4) in einem Zwischenraum (3) gelagert ist, der mit der Austragsöffnung (23) der Mischvorrichtung (10) verbindbar ist und in den das Leitungsmittel (122) endet.

3. Knochenzementsystem (100) nach wenigstens einem der vorherigen Ansprüche, **dadurch gekennzeichnet, dass** das Siebelement (4) eine Maschenweite von kleiner als 30 µm hat, insbesondere eine Maschenweite zwischen 5 µm und 25 µm.

4. Knochenzementsystem (100) nach wenigstens einem der vorherigen Ansprüche, **dadurch gekennzeichnet, dass** das Verhältnis der Fläche der Durchgangsöffnung (2) zur Fläche des Siebelementes (4) zwischen 1 zu 4 und 1 zu 20 ist.

5. Knochenzementsystem (100) nach wenigstens einem der vorherigen Ansprüche, **dadurch gekennzeichnet, dass** der Abstand zwischen der Blende (1) und dem Siebelement (4) zwischen 2 mm bis 10 mm ist.

6. Knochenzementsystem (100) nach wenigstens einem der vorherigen Ansprüche, **dadurch gekennzeichnet, dass** die Durchgangsöffnung (2) kreisförmig, oval, sternförmig oder schlitzförmig ausgebildet ist, insbesondere dass die Blende (1) trichterförmig verläuft.

7. Knochenzementsystem (100) nach wenigstens einem der vorherigen Ansprüche, **dadurch gekennzeichnet, dass** in dem Mischzylinder (20) ein Mischkolben (21) angeordnet ist, wobei der Mischkolben (21) mittels einer an einem ersten Zylinderende (30) gedichtet herausgeführten Betätigungsstange (50) axial bewegbar ist.

8. Knochenzementsystem (100) nach wenigstens einem der vorherigen Ansprüche, **dadurch gekennzeichnet, dass** ein Kolbensystem (40) axial in den Mischzylinder (20) eindrückbar ist, um einen aus dem Knochenzementpulver und dem Monomer gemischten Knochenzement durch die Austragsöffnung (23) auszutragen.

9. Knochenzementsystem (100) nach wenigstens einem der vorherigen Ansprüche, **dadurch gekennzeichnet, dass** das Vorratselement (110) ein Ventilmittel (115) aufweist, um einen Ausfluss des Monomers aus dem Vorratsbehälter (112) zu steuern und/oder auszulösen.

10. Knochenzementsystem (100) nach wenigstens einem der vorherigen Ansprüche, **dadurch gekennzeichnet, dass** das Knochenzementsystem (100) ein Basiselement (120) aufweist, wobei das Basiselement (120) die Mischvorrichtung (10) und den Vorratsbehälter (112) lagert.

## Claims

1. Bone cement system (100) with a mixing device (10) for the mixing and dispensing of bone cement, a reservoir container (112) for a monomer and a conduit means (122), whereby
the mixing device (10) comprises the mixing cylinder (20), the mixing cylinder (20) stores a bone cement powder,
the monomer can be guided from the reservoir container (112) into the mixing cylinder (20) by means of the conduit means (122),
a screen element (4) is arranged between the reservoir container (112) and the mixing device (10) in order to prevent the bone cement powder from flowing from the mixing cylinder (20) into the conduit means (122),
the mixing device (10) comprises a dispensing opening (23) for dispensing a bone cement mixed from the bone cement powder and the monomer, **characterised in that**
the dispensing opening (23) comprises a panel (1) with at least one feed-through opening (2), and
a ratio of a surface area of the feed-through opening (2) to the surface area of the screen element (4) is at least 1 to 3, and a distance between the panel (1) and the screen element (4) is at least 1 mm.

2. Bone cement system (100) according to claim 1, **characterised in that** the screen element (4) is supported in a clearance (3) that can be connected to the dispensing opening (23) of the mixing device (10), and in which the conduit means (122) terminates.

3. Bone cement system (100) according to at least one of the preceding claims, **characterised in that** the screen element (4) has a mesh width of less than 30 µm, in particular a mesh width between 5 µm and 25 µm.

4. Bone cement system (100) according to at least one of the preceding claims, **characterised in that** the ratio of the surface area of the feed-through opening (2) to the surface area of the screen element (4) is between 1 to 4 and 1 to 20.

5. Bone cement system (100) according to at least one of the preceding claims, **characterised in that** the distance between the panel (1) and the screen element (4) is between 2 mm to 10 mm.

6. Bone cement system (100) according to at least one of the preceding claims, **characterised in that** the feed-through opening (2) is provided to be circular, oval, star-shaped or slit-shaped, in particular **in that** the panel (1) extends to be funnel-shaped.

7. Bone cement system (100) according to at least one of the preceding claims, **characterised in that** a mixing plunger (21) is arranged in the mixing cylinder (20), whereby the mixing plunger (21) can be moved axially by means of an actuation rod (50) that is guided out, in sealed manner, at a first cylinder end (30).

8. Bone cement system (100) according to at least one of the preceding claims, **characterised in that** a plunger system (40) can be pushed axially into the mixing cylinder (20) in order to dispense through the dispensing opening (23) a bone cement that has been mixed from the bone cement powder and the monomer.

9. Bone cement system (100) according to at least one of the preceding claims, **characterised in that** the reservoir element (110) comprises a valve means (115) for controlling and/or triggering an outward flow of the monomer from the reservoir container (112).

10. Bone cement system (100) according to at least one of the preceding claims, **characterised in that** the bone cement system (100) comprises a base element (120), whereby the mixing device (10) and the reservoir container (112) are stored in the base element (120).

## Revendications

1. Système de ciment osseux (100) avec un dispositif de mélange (10) pour mélanger et distribuer du ciment osseux, un réservoir (112) pour un monomère et un moyen de transmission (122), dans lequel le dispositif de mélange (10) présente un cylindre de mélange (20),
le cylindre de mélange (20) stocke une poudre de ciment osseux,
le monomère peut être conduit du réservoir (112) dans le cylindre de mélange (20) au moyen du moyen de transmission (122),
un élément de tamis (4) est disposé entre le réservoir (112) et le dispositif de mélange (10) pour empêcher un afflux de la poudre de ciment osseux du cylindre de mélange (20) dans le moyen de transmission (122),
le dispositif de mélange (10) présente une ouverture de distribution (23) pour distribuer un ciment osseux mélangé à partir de la poudre de ciment osseux et du monomère,
**caractérisé en ce que**
l'ouverture de distribution (23) présente un écran (1) avec au moins une ouverture de passage (2), et
un rapport d'une surface de l'ouverture de passage (2) sur la surface de l'élément de tamis (4) est d'au moins 1 sur 3 et un écart entre l'écran (1) et l'élément de tamis (4) est d'au moins 1 mm.

2. Système de ciment osseux (100) selon la revendication 1, **caractérisé en ce que** l'élément de tamis (4) est logé dans un espace intermédiaire (3) qui peut être connecté à l'ouverture de distribution (23) du dispositif de mélange (10) et dans lequel se termine le moyen de transmission (122).

3. Système de ciment osseux (100) selon au moins une des revendications précédentes, **caractérisé en ce que** l'élément de tamis (4) a une largeur de maille inférieure à 30 µm, notamment une largeur de maille comprise entre 5 µm et 25 µm.

4. Système de ciment osseux (100) selon au moins une des revendications précédentes, **caractérisé en ce que** le rapport de la surface de l'ouverture de passage (2) sur la surface de l'élément de tamis (4) est compris entre 1 sur 4 et 1 sur 20.

5. Système de ciment osseux (100) selon au moins une des revendications précédentes, **caractérisé en ce que** l'écart entre l'écran (1) et l'élément de tamis (4) est compris entre 2 mm et 10 mm.

6. Système de ciment osseux (100) selon au moins une des revendications précédentes, **caractérisé en ce que** l'ouverture de passage (2) est réalisée de manière circulaire, ovale, en forme d'étoile ou en forme de fente, notamment que l'écran (1) s'étend en forme d'entonnoir.

7. Système de ciment osseux (100) selon au moins une des revendications précédentes, **caractérisé en ce qu'**un piston de mélange (21) est disposé dans le cylindre de mélange (20), dans lequel le piston de mélange (21) est mobile axialement au moyen d'une tige d'actionnement (50) sortie de manière hermétique contre une première extrémité de cylindre (30).

8. Système de ciment osseux (100) selon au moins une des revendications précédentes, **caractérisé en ce qu'**un système de piston (40) peut être enfoncé axialement dans le cylindre de mélange (20) pour distribuer du ciment osseux mélangé à partir de la poudre de ciment osseux et du monomère à travers l'ouverture de distribution (23).

9. Système de ciment osseux (100) selon au moins une des revendications précédentes, **caractérisé en ce que** l'élément de réserve (110) présente un moyen de soupape (115) pour commander et/ou déclencher un écoulement du monomère du réservoir (112).

10. Système de ciment osseux (100) selon au moins une des revendications précédentes, **caractérisé en ce que** le système de ciment osseux (100) présente un élément de base (120), dans lequel l'élément de base (120) loge le dispositif de mélange (10) et le réservoir (112).
